(19) Europäisches Patentamt
European Patent Office
Office européen des brevets

(11) Veröffentlichungsnummer : **0 008 097**
**B1**

(12) # EUROPÄISCHE PATENTSCHRIFT

(45) Veröffentlichungstag der Patentschrift : **21.10.81**

(51) Int. Cl.³ : **C 07 D209/08**, C 07 D209/96, C 07 D209/60// C07D263/38, C07D263/52

(21) Anmeldenummer : **79102806.1**

(22) Anmeldetag : **04.08.79**

(54) **Verfahren zur Herstellung von Indoleninen.**

(30) Priorität : **07.08.78 DE 2834607**

(43) Veröffentlichungstag der Anmeldung : **20.02.80 (Patentblatt 80/04)**

(45) Bekanntmachung des Hinweises auf die Patenterteilung : **21.10.81 Patentblatt 81/42**

(84) Benannte Vertragsstaaten : **CH DE FR GB IT**

(56) Entgegenhaltungen :

**TETRAHEDRON, Vol. 24, Seiten 6663-6669, 1968 GB**
**B. ZEEH : «Heterocyclen aus Isocyaniden-IV¹»**

(73) Patentinhaber : **BASF Aktiengesellschaft**
**Carl-Bosch-Strasse 38**
**D-6700 Ludwigshafen (DE)**

(72) Erfinder : **Laas, Harald, Dr.**
**Woehlerstrasse 14**
**D-6701 Maxdorf (DE)**
Erfinder : **Nissen, Axel, Dr.**
**Panoramastrasse 51**
**D-6906 Leimen (DE)**
Erfinder : **Opgenorth, Hans-Joachim, Dr.**
**Hanns-Fay-Strasse 1**
**D-6710 Frankenthal (DE)**
Erfinder : **Scheuermann, Horst, Dr.**
**Bexbacher Strasse 41**
**D-6700 Ludwigshafen (DE)**
Erfinder : **Mueller, Hans-Richard, Dr.**
**An der Tuchbleiche 9**
**D-6712 Bobenheim-Roxheim (DE)**
Erfinder : **Schulte, Wolfgang, Dr.**
**Barbarossastrasse 9**
**D-6733 Hassloch (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 008 097 B1

Imprimerie Jouve, 17, rue du Louvre, 75001 Paris, France

# Verfahren zur Herstellung von Indoleninen

Die Erfindung betrifft ein Verfahren zur Herstellung von Verbindungen der allgemeinen Formel I

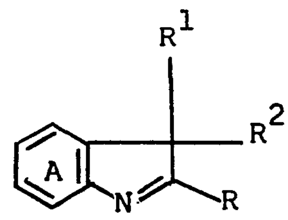

(I),

in der

R, $R^1$ und $R^2$ $C_1$-bis $C_9$-Alkyl oder -Alkenyl bedeuten und wobei $R^1$ und $R^2$ zusammen auch Pentamethylen sein können und der Ring A noch durch Chlor, Brom, Nitro, Cyan, $C_1$- bis $C_4$-Alkoxycarbonyl, Methylsulfonyl, Äthylsulfonyl, Methoxy, Äthoxy oder $C_1$-bis $C_4$-Alkyl oder einen anellierten Benzring substituiert sein kann, das dadurch gekennzeichnet ist, daß man Verbindungen der Formel II

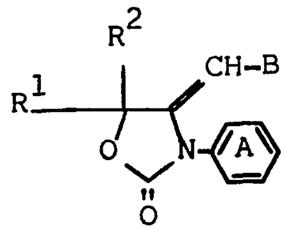

(II)

in der

B Wasserstoff oder $C_1$-bis $C_8$-Alkyl oder -Alkenyl ist, in Gegenwart von Lewissäuren und Halogeniden erhitzt. Einzelne Reste R, $R^1$ und $R^2$ sind beispielsweise Äthyl, Propyl, Butyl, Hexyl, Octyl, Nonyl, Allyl und insbesondere Methyl.

Als Substituenten für den Ring A kommen neben den bereits genannten z.B. Methoxy-, Äthoxy- oder Butoxycarbonyl, Methyl, Äthyl, Propyl oder Butyl in Betracht.

Als Lewissäuren für die Umsetzung eignen sich z.B. Zinn-II- oder Zinn-IV-chlorid, Titantetrachlorid, Bortrifluorid, Aluminiumchlorid oder Eisen-III-chlorid und insbesondere Zinkchlorid. Die Gegenwart von Halogeniden ist für die Umsetzung anscheinend erforderlich ; weitere als Zusatz geeignete Halogenide sind beispielsweise NaCl, KCl, LiCl, $MgCl_2$, $CaCl_2$, $BaCl_2$, NaBr, NaJ, LiJ oder KJ. Bevorzugt ist die Verwendung von halogenhaltigen Lewissäuren, insbesondere Zinkchlorid. Eine Kombination von beispielsweise nicht halogenhaltigen Zinksalzen und Halogeniden kann manchmal ebenfalls vorteilhaft sein. Besonders bevorzugt ist die Kombination Zinkchlorid/Lithiumchlorid im Molverhältnis von ungefähr 1 : 0,5 bis 1 : 4, vorzugsweise 1 : 2.

Zweckmäßigerweise führt man das erfindungsgemäße Verfahren so durch, daß man die Verbindungen der Formel II in Gegenwart oder Abwesenheit von Lösungsmitteln mit den Lewissäuren und Halogeniden auf Temperaturen von 150 bis 250 °C, vorzugsweise 180 bis 220 °C, erhitzt. Die Umsetzung zu den Verbindungen der Formel I erfolgt unter $CO_2$-Abspaltung und ist in der Regel nach 2 bis 20 Stunden beendet.

Pro Mol an Verbindung II verwendet man zweckmäßigerweise 1 bis 50 Molprozent, vorzugsweise 5 bis 15 Molprozent, and Lewissäuren plus gegebenenfalls Halogenid.

Als Lösungsmittel für die Umsetzung kommen insbesondere hochsiedende, unter den Reaktionsbedingungen inerte Verbindungen in Betracht, beispielsweise seien Silikonöle oder vorzugsweise Mineralöle genannt, die selbstverständlich nach Abtrennung des Reaktionsproduktes mit dem Katalysator zurückgeführt werden können.

Verbindungen der Formel II erhält man aux Verbindungen der Formel III

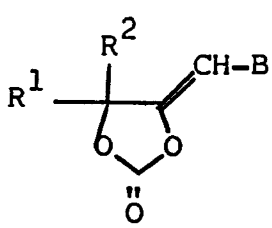

(III)

durch Umsetzung mit Aminen der Formel IV

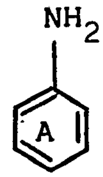

(IV)

bei erhöhter Temperatur unter Entfernung des Wassers. Die Amine der Formel IV werden zweckmäßigerweise im Überschuß verwendet, bevorzugt sind Molverhältnisse von 1 : 1,5 bis 1 : 3.

Eine besonders bevorzugte Variante des erfindungsgemäßen Verfahrens besteht darin, daß man die Herstellung der Verbindung der Formel II vorschaltet und diese ohne Isolierung in einem Eintopfverfahren zu den Verbindungen der Formel I umsetzt.

Von besonderer Bedeutung ist das erfindungsgemäße Verfahren zur Herstellung von Verbindungen der Formel Ia

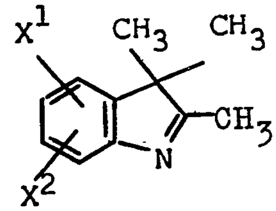

Ia,

in der

$X^1$ Wasserstoff, Chlor, Methyl oder Methoxy und

$X^2$ Wasserstoff, Chlor oder Methyl sind.

Gegenüber dem in der GB-A-2003139 beschriebenen Verfahren des Standes der Technik bietet das erfindungsgemäße Verfahren große Vorteile bei dem als Ausgangsmaterial 3-Chlor-3-methylbutanon-2 benötigt wird.

Die erfindungsgemäß hergestellten Verbindungen eignen sich insbesondere als Zwischenprodukte zur Herstellung basischer Cyanin- und Azacyaninfarbstoffe.

In den folgenden Beispielen beziehen sich Angaben über Teile und Prozente, sofern nicht anders vermerkt, auf das Gewicht.

Beispiel 1

Zu einem unter Rückfluß siedenden Gemisch von 1 116 Teilen Anilin, 400 Teilen Mineralöl und 80 Teilen Zinkchlorid wird unter Rühren innerhalb von 6 Stunden ein Gemisch von 1 024 Teilen 4,4-Dimethyl-3-methylen-dioxolanon und 372 Teilen Anilin zugetropft. Parallel zur $CO_2$-Entwicklung wird Wasser abgespalten, das kontinuierlich aus dem Reaktionsgemisch herausdestilliert. Nach Beendigung des Zutropfens wird noch weitere 10 Stunden bei 210 °C gerührt. Bei der anschließenden Destillation werden 992 Teile 2-Methyl-3,3-dimethyl-indolenin erhalten (78 % der Theorie).

Beispiel 2

Ein Gemisch von 128 Teilen 4,4-Dimethyl-3-methylen-dioxolanon, 112 Teilen Anilin, 100 Teilen o-Xylol und 5 Teilen Zinkchlorid wird am Wasserauskreiser erhitzt. Es werden in 12 Stunden 18 Teile Wasser ausgekreist. 198 Teile 1-Phenyl-4,4-dimethyl-5-methylen-oxazolidinon werden isoliert (97,6 % der Theorie). Schmelzpunkt 115 bis 116 °C.

Beispiel 3

203 Teile 1-Phenyl-4,4-dimethyl-5-methylen-oxazolidinon werden mit 20 Teilen $ZnCl_2$ gut durchmischt und dann auf 210 °C erhitzt. Die $CO_2$-Abspaltung ist nach 8 Stunden beendet. Es werden 97 Teile Indolenin isoliert (61,6 % der Theorie).

Beispiel 4

203 Teile 1-Phenyl-4,4-dimethyl-5-methylen-oxazolidinon werden mit 20 Teilen $ZnCl_2$ zusammen in 100 Teilen eines hochsiedenden Mineralöls aufgeschwämmt und dann 8 Stunden auf 210 °C erhitzt. Bei der nachfolgenden Destillation werden 113 Teile Indolenin erhalten (71,2 %).

Gemäß Beispiel 2 können nachfolgende Oxazolidinonderivate hergestellt werden:

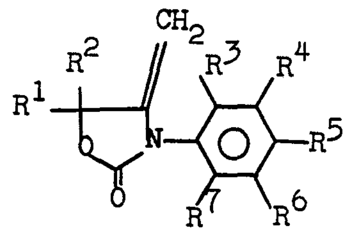

| Beispiel | $R_1$ | $R_2$ | $R_3$ | $R_4$ | $R_5$ | $R_6$ | $R_7$ | Ausbeute | Schmelzpunkt °C |
|---|---|---|---|---|---|---|---|---|---|
| 5 | $CH_3$ | $C_2H_5$ | H | H | H | H | H | 47,4 % | 131-132 |
| 6 | $CH_3$ | $CH_3$ | H | Cl | Cl | H | H | 38,7 % | 118-119 |
| 7 | $CH_3$ | $CH_3$ | H | H | Cl | H | H | 40,3 % | 114-117 |
| 8 | $CH_3$ | $CH_3$ | annel Benzring | | H | H | H | 28,3 % | 122 |
| 9 | $CH_3$ | $CH_3$ | H | H | $OC_2H_5$ | H | H | 47,2 % | 68 |
| 10 | $CH_3$ | $CH_3$ | H | H | $CH_3$ | H | H | 45,3 % | 205 |
| 11 | Cyclohexyl | | H | H | H | H | H | 74,2 % | 148-150 |
| 12 | $CH_3$ | $CH_3$ | H | H | $CO_2C_2H_5$ | H | H | 35,3 % | 91- 93 |

Gemäß Beispiel 1, 3 oder 4 können die unter den Beispielen 5-12 aufgeführten Oxazolidinon-Derivate in die entsprechenden Indoleninderivate überführt werden.

Die Verbindungen der Beispiele 13 bis 19 wurden analog Beispiel 4 hergestellt.

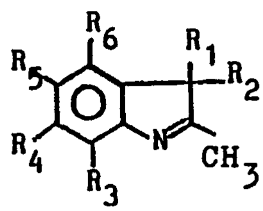

| Beispiel | $R_1$ | $R_2$ | $R_3$ | $R_4$ | $R_5$ | $R_6$ | Ausbeute | Siedepunkt °C |
|---|---|---|---|---|---|---|---|---|
| 13 | $CH_3$ | $C_2H_5$ | H | H | H | H | 29,5 % | 122-124/2 |
| 14 | $CH_3$ | $CH_3$ | H | Cl | Cl | H | 42,3 % | 122-113/1 |
| 15 | $CH_3$ | $CH_3$ | H | H | Cl | H | 29,7 % | 80- 82/0.3 |
| 16 | $CH_3$ | $CH_3$ | annel Benzring | | H | H | 39,3 % | 125/0,5 |
| 17 | $CH_3$ | $CH_3$ | H | H | $OC_2H_5$ | H | 50,7 % | 110-112/1,5 |
| 18 | $CH_3$ | $CH_3$ | H | H | $CH_3$ | H | 11,6 % | 77- 78/1 |
| 19 | $CH_3$ | $CH_3$ | H | H | $CO_2C_2H_5$ | H | 15,3 % | 102-104/1,5 |

Beispiel 20

Zu einem unter Rückfluß siedenden Gemisch aus 1 116 Teilen Anilin, 400 Teilen Mineralöl, 80 Teilen Zinkchlorid und 55 Teilen Lithiumchlorid, wird innerhalb von 6 Stunden ein Gemisch aus 1024 Teilen 4,4-Dimethyl-3-methylendioxolanon und 372 Teilen Anilin zudosiert. Parallel zur $CO_2$-Entwicklung wird Wasser abgespalten, das zusammen mit Anilin aus dem Reaktionsgemisch herausdestilliert und nach Kühlung an einem kontinuierlich arbeitendem Wasserauskreiser vom Anilin abgetrennt wird. Das Anilin wird in das Reaktionsgemisch zurückgeführt. Nach Beendigung der Zudosierung wird zur Vervollständigung der Reaktion noch weitere 10 Stunden bei 205 bis 210 °C gerührt. Bei der anschließenden Destillation werden 1 102 Teile 2-Methyl-3,3-dimethyl-indolenin erhalten. (86,6 % der Theorie).

**Ansprüche**

1. Verfahren zur Herstellung von Indoleninen der allgemeinen Formel I

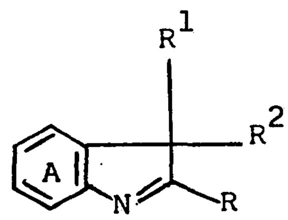

(I),

in der

R, $R^1$ und $R^2$ $C_1$- bis $C_9$-Alkyl oder -Alkenyl bedeuten und wobei $R^1$ und $R^2$ zusammen auch Pentamethylen sein können und der Ring A noch durch Chlor, Brom, Nitro, Cyan, $C_1$- bis $C_4$-Alkoxycarbonyl, Methylsulfonyl, Äthylsulfonyl, Methoxy, Äthoxy oder $C_1$- bis $C_4$-Alkyl oder einen annellierten Benzring substituiert sein kann, das dadurch gekennzeichnet ist, daß man Verbindungen der Formel II

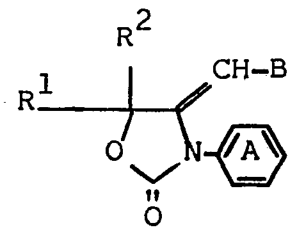

(II)

in der

B Wasserstoff oder $C_1$- bis $C_8$-Alkyl oder -Alkenyl ist, in Gegenwart von Lewissäuren und Halogeniden erhitzt.

2. Verfahren gemäß Anspruch 1, dadurch gekennzeichnet, daß man Verbindungen der Formel

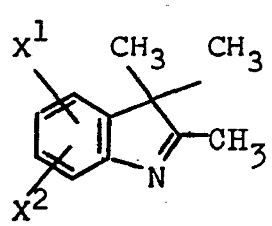

herstellt, in der

$X^1$ Wasserstoff, Chlor, Methyl oder Methoxy und

$X^2$ Wasserstoff, Chlor oder Methyl sind.

3. Verfahren gemäß Anspruch 1, dadurch gekennzeichnet, daß man ein Gemisch von Zinkchlorid/Lithiumchlorid als Lewissäure und Halogenid verwendet.

4. Verfahren gemäß Anspruch 1, dadurch gekennzeichnet, daß man durch Umsetzung von Verbindungen der Formel III

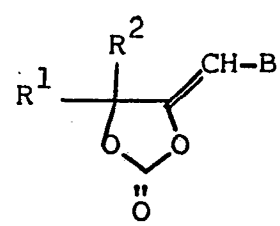

(III)

mit Aminen der Formel IV

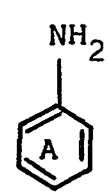

(IV)

bei Erhitzung unter Entfernen des Wassers erhaltene Verbindungen der Formel II ohne vorherige Isolierung zu den Verbindungen der Formel I gemäß Anspruch 1 weiter umsetzt.

## Claims

1. A process for the preparation of an indolenine of the general formula I

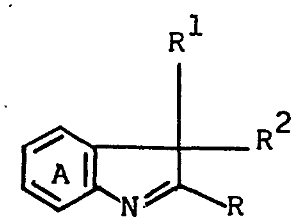

(I)

where

R, $R^1$ and $R^2$ are $C_1$-$C_8$-alkyls or -alkenyls, and $R^1$ and $R^2$ together may be pentamethylene, and the ring a may be additionally substituted by chlorine, bromine, nitro, cyano, $C_1$-$C_4$-alkoxycarbonyl, methylsulfonyl, ethylsulfonyl, methoxy, ethoxy or $C_1$-$C_4$-alkyl or a fused benzene ring, which is characterized in that a compound of the formula II

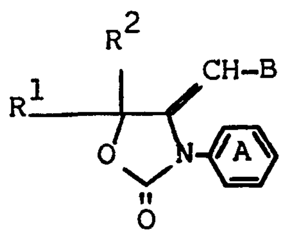

(II)

where

B is hydrogen or $C_1$-$C_8$-alkyl or -alkenyl, is heated in the presence of a Lewis acid and of a halide.

2. A process as claimed in claim 1, characterized in that a compound of the formula

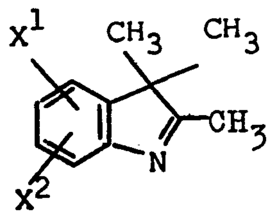

where

$X^1$ is hydrogen, chlorine, methyl or methoxy, and $X^2$ is hydrogen, chlorine or methyl, is prepared.

3. A process as claimed in claim 1, characterized in that a mixture of zinc chloride and lithium chloride is used as the Lewis acid and halide.

4. A process as claimed in claim 1, characterized in that a compound of the formula II, obtained by reacting a compound of the formula III

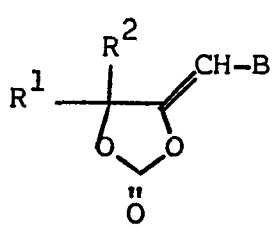

(III)

with an amine of the formula IV

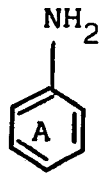

(IV)

by heating while removing the water, is further reacted, without prior isolation, to give the compound of the formula I as claimed in claim 1.

**Revendications**

1. Procédé pour la préparation d'indolénines de formule générale I

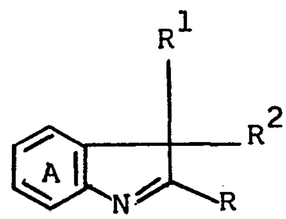

(I)

dans laquelle

R, $R^1$ et $R^2$ représentent des radicaux alcoyle ou alcényle à 1-9 C, $R^1$ et $R^2$ pouvant également former ensemble un pentaméthylène et le noyau A pouvant être encore substitué par un chlore, un brome, un nitro, un cyano, un alcoxycarbonyle à 1-4 C, un méthylsulfonyle, un éthylsulfonyle, un méthoxy, un éthoxy, un alcoyle à 1-4 C ou un noyau benzénique condensé, caractérisé en ce qu'on chauffe des composés de formule II

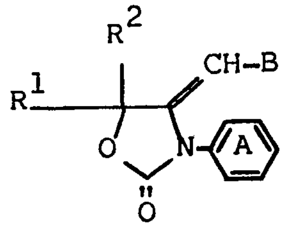

(II)

dans laquelle

B est un hydrogène ou un alcoyle ou alcényle à 1-8 C, en présence d'acides de Lewis et d'halogénures.

2. Procédé selon la revendication 1, caractérisé en ce qu'on prépare des composés de formule

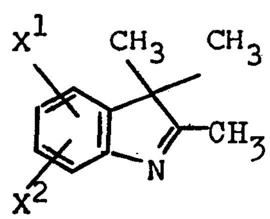

dans laquelle

$X^1$ représente un hydrogène, un chlore, un méthyle ou un méthoxy et $X^2$ un hydrogène, un chlore ou un méthyle.

3. Procédé selon la revendication 1, caractérisé en ce qu'on utilise, en tant qu'acide de Lewis et halogénure, un mélange de chlorure de zinc/chlorure de lithium.

4. Procédé selon la revendication 1, caractérisé en ce qu'on retransforme en les composés de formule I selon la revendication 1, sans isolement préalable, des composés de formule II obtenus par réaction de composés de formule III

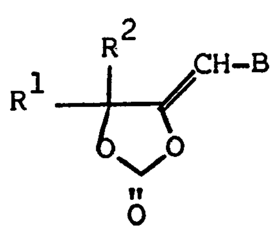

(III)

avec des amines de formule IV

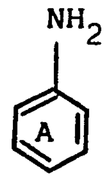

(IV)

à température élevée et avec élimination de l'eau.